# EUROPÄISCHE PATENTSCHRIFT

(11) **EP 3 173 130 B1**
(45) Veröffentlichungstag und Bekanntmachung des Hinweises auf die Patenterteilung: **08.01.2020**
(21) Anmeldenummer: 16196696.5
(22) Anmeldetag: 01.11.2016
(51) Int. Cl.: A61Q 17/04, A61K 8/29, A61K 8/37, A61K 8/40, A61K 8/34

(54) **TITANDIOXID-HALTIGES SONNENSCHUTZMITTEL MIT OCTOCRYLEN**
SUNSCREEN COMPRISING TITANIUM DIOXIDE AND OCTOCRYLENE
COMPOSITION ANTI-SOLAIRE COMPRENANT DU DIOXIDE DE TITANIUM ET DE L'OCTOCRYLENE

(30) Priorität: 25.11.2015 DE 102015223261
(43) Veröffentlichungstag der Anmeldung: 31.05.2017
(73) Patentinhaber: Beiersdorf AG, 20253 Hamburg (DE)
(72) Erfinder: Eitrich, Anja, 25337 Kölln-Reisiek (DE); Sprock, Sarah, 22297 Hamburg (DE); Schade, Juliane, 22529 Hamburg (DE); Göddertz, Dominik, 22763 Hamburg (DE); Eisert, Anja, 22085 Hamburg (DE); Reiter, Katharina, 21357 Barum (DE)

(56) Entgegenhaltungen:
- WO-A2-2011/061133
- DE-A1-102014 207 602
- US-A1- 2006 104 923
- US-A1- 2009 041 817
- MARIA EUGENIA CARLOTTI ET AL: "Role of particle coating in controlling skin damage photoinduced by titania nanoparticles", FREE RADICAL RESEARCH, Bd. 43, Nr. 3, 1. Januar 2009 (2009-01-01) , Seiten 312-322, XP055329199, GB ISSN: 1071-5762, DOI: 10.1080/10715760802716633
- E. GILBERT ET AL: "Commonly used UV filter toxicity on biological functions: review of last decade studies", INTERNATIONAL JOURNAL OF COSMETIC SCIENCE, 15. Januar 2013 (2013-01-15), Seiten n/a-n/a, XP055063284, ISSN: 0142-5463, DOI: 10.1111/ics.12030
- "Use of an agent for screening out light radiation with a wavelength ranging from 340 to 400nm", IP.COM JOURNAL, IP.COM INC., WEST HENRIETTA, NY, US, 5. Februar 2007 (2007-02-05), XP013117867, ISSN: 1533-0001

## Beschreibung

Die vorliegende Erfindung betrifft eine kosmetische Zubereitung enthaltend 2-Ethylhexyl-2-cyano-3,3-diphenylacrylat (Octocrylen), Ethanol und Titandioxid in der Kristallstruktur Rutil mit einer Primärpartikelgröße von 2 -100 nm.

Der Trend weg von der vornehmen Blässe hin zur "gesunden, sportlich braunen Haut" ist seit Jahren ungebrochen. Um diese zu erzielen setzen die Menschen ihre Haut der Sonnenstrahlung aus, da diese eine Pigmentbildung im Sinne einer Melaninbildung hervorruft. Die ultraviolette Strahlung des Sonnenlichtes hat jedoch auch eine schädigende Wirkung auf die Haut. Neben der akuten Schädigung (Sonnenbrand) treten Langzeitschäden wie ein erhöhtes Risiko an Hautkrebs zu erkranken bei übermäßiger Bestrahlung mit Licht aus dem UVB-Bereich (Wellenlänge: 280-320 nm) auf. Die übermäßige Einwirkung der UVB- und UVA-Strahlung (Wellenlänge: 320-400 nm) führt darüber hinaus zu einer Schwächung der elastischen und kollagenen Fasern des Bindegewebes. Dies führt zu zahlreichen phototoxischen und photoallergischen Reaktionen und hat eine vorzeitige Hautalterung zur Folge.

Zum Schutz der Haut wurden daher eine Reihe von Lichtschutzfiltersubstanzen entwickelt, die in kosmetischen Zubereitungen eingesetzt werden können. Diese UVA- und UVB-Filter sind in den meisten Industrieländern in Form von Positivlisten wie dem Anlage 7 der Kosmetikverordnung zusammengefasst.

Die Vielzahl an kommerziell erhältlichen Sonnenschutzmitteln darf jedoch nicht darüber hinwegtäuschen, dass diese Zubereitungen des Standes der Technik eine Reihe von Nachteilen aufweisen.

Ein vielfach in Tagespflegeprodukten und Sonnenschutzmitteln eingesetzter UV-Filter ist die Verbindung 2-Ethylhexyl-2-cyano-3,3-diphenylacrylat (Octocrylen), die neben ihren UV-Filtereigenschaften im UV-B-Bereich vor allem dazu verwendet wird den UV-A-Filter 4-(tert.-Butyl)-4'-methoxydibenzoylmethan gegen photochemischen Abbau zu stabilisieren. Octocrylen wird dabei in der Regel in Kombination mit weiteren UV-Filtern eingesetzt, beispielsweise mit Titandioxid.

Nachteilig am Stande der Technik ist dabei der Umstand, dass Octocrylen in Gegenwart von Ethanol in der Regel dazu führt, dass bei den Zubereitungen im Verlauf ihrer Lagerung sowie bei der Anwendung auf der Haut nach einer gewissen Zeit ein "Fehlgeruch" auftritt. Der entstehende Geruch erinnert entfernt an die aus der Küche bekannte Maggi-Stammwürze oder auch an den Geruch von Liebstöckel. Ursache für diesen Fehlgeruch ist die Bildung von Sotolon (3-Hydroxy-4,5-dimethyl-5H-furan-2-on), welches die folgende Struktur aufweist: 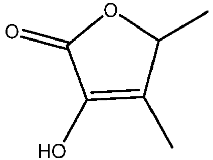 Der Reaktionsmechanismus, welcher zur Entstehung von 3-Hydroxy-4,5-dimethyl-5H-furan-2-on (im Folgenden Sotolon genannt) führt, ist bis heute unbekannt und unverstanden.

Eine Möglichkeit, den Fehlgeruch zu vermeiden, besteht natürlich darin, auf den Einsatz von Ethanol zu verzichten. Ethanol wird jedoch zunehmend dazu verwendet, die bei den Verbrauchern so beliebten "Konservierungsmittel freien" kosmetischen Zubereitungen vor mikrobiellem Befall zu schützen. Darüber hinaus bildet Ethanol aufgrund seiner Lösungseigenschaften, seiner sensorischen Eigenschaften sowie seines geringen Preises die ideale Grundlage für transparente Sprayzubereitungen, beispielsweise Sonnenschutzsprays.

Nach dem Stand der Technik versucht man daher bei ethanolischen Zubereitungen, die geruchliche Fehlnote des Sotolons durch den Einsatz entsprechend hoher Mengen an Parfümstoffen zu maskieren. Diese Lösung hat jedoch den Nachteil, dass sich die Parfümstoffe im Laufe der Zeit aus der Zubereitung heraus verflüchtigen, während die Konzentration an Sotolon im Verlaufe der Zeit ansteigt. Darüber hinaus gibt es auch bei Kosmetika einen wachsenden Bedarf an sogenannten "parfümfreien" Zubereitungen, bei denen eine Geruchsmaskierung dann nicht möglich ist.

Im Stande der Technik sind eine Vielzahl von Sonnenschutzmitteln bekannt, die neben Octocrylen und Ethanol auch mikronisiertes Titandioxid enthalten. Dabei werden in der Regel Titandioxide eingesetzt, die ganz oder teilweise in der Kristallstruktur Anatas vorliegen, beispielsweise das Titandioxid mit dem Handelsnamen Tego Sun T 805 von Evonik, welches eine Mischung aus den Kristallstrukturen Anatas/Rutil von etwa 80:20 aufweist. Zubereitungen mit Titandioxid in Anatas-Struktur haben dabei den Vorteil, transparenter zu sein als vergleichbare Zubereitungen mit Titandioxid in Rutil-Struktur.

Nachteilig am Stande der Technik ist jedoch der Umstand, dass die Zubereitungen enthaltend Titandioxid in Anatas-Struktur keinen Einfluss auf die Bildung des Fehlgeruches durch Sotolon hat.

Es war daher die Aufgabe der vorliegenden Erfindung, eine kosmetische Zubereitung (insbesondere ein Tagespflegeprodukt oder ein Sonnenschutzmittel) enthaltend Octocrylen, Titandioxid und Ethanol zu entwickeln, bei dem die Bildung eines Fehlgeruches und insbesondere die Bildung des Sotolons unterdrückt wird.

Nachteilig am Stande der Technik ist darüber hinaus der Umstand, dass viele Titandioxid enthaltende Sonnenschutzmittel bei der Lagerung über einen längeren Zeitraum zur Bildung von Gasbläschen neigen. Diese Gasbildung (Wasserstoff) tritt dabei auch bei beschichteten Titandioxiden immer wieder auf. Es war daher die Aufgabe der vorliegenden Erfindung, ein Sonnenschutzmittel auf der Basis von mikronisiertem Titandioxid zu entwickeln, bei dem die Gasbildung während der Lagerung wirkungsvoll unterdrückt ist.

Gelöst werden die Aufgaben durch eine kosmetische Zubereitung gemäß Anspruch 1.

Es ist erfindungsgemäß, wenn die erfindungsgemäße Zubereitung ausschließlich Titandioxid in der Kristallstruktur Rutil enthält. Dabei gilt erfindungsgemäß ein Titandioxid dann als ausschließlich in der Kristallstruktur vorliegend, wenn der Gehalt an anderen Kristallstrukturen in der Summe 1 Gewichts-%, bezogen auf die Gesamtmenge an Titandioxid, nicht überschreitet.

Es ist erfindungsgemäß bevorzugt, wenn die Primärpartikelgröße des erfindungsgemäßen Titandioxides im Bereich zwischen 5 und 50 nm liegt.

Zwar kennt der Stand der Technik die Dokumente WO 2011/061133 A2, betreffend doppelt beschichtete Titandioxide zur Erhöhung der Wasserfestigkeit, US 2009/041817 A1, betreffend W/O Emulsionen mit Titandioxid und Octocrylen sowie US 2006/104923 A1, betreffend ethanolische Sonnenschutzmittel mit fluorierten Alkylethern, doch konnten diese Schriften nicht den Weg zur vorliegenden Erfindung weisen.

Es ist erfindungsgemäß, wenn das Titandioxid mit Silica (Siliziumdioxid und/oder Kieselsäure) beschichtet ist und wenn das mit Silica beschichtete Titandioxid auf der äußeren Seite der Silica-Schicht (d.h. auf der dem Titandioxid-abgewandten Seite) eine Schicht aus Dimethicone aufweist.

Es ist erfindungsgemäß vorteilhaft, wenn die sekundäre Partikelgröße des Titandioxid in der Kristallstruktur Rutil mit einer Primärpartikelgröße von 2 -100 nm zwischen 0,05 und 50 µm beträgt. Dabei ist der Bereich von 0,1 bis 1 µm erfindungsgemäß bevorzugt.

Die primäre und sekundäre Partikelgröße entnimmt der Fachmann folgender Literaturstelle: SCCS/1516/13 Opinion on Titanium Dioxide (nano form) Colipa No S75 der Cosmetics Europe personal care association.

Es ist vorteilhaft im Sinne der vorliegenden Erfindung, wenn die Zubereitung Octocrylen in einer Konzentration von 0,5 bis 9,5 Gewichts-%, bezogen auf das Gesamtgewicht der Zubereitung, enthält. Dabei wird ein Gehalt von 3 bis 9 Gewichts-%, bezogen auf das Gesamtgewicht der Zubereitung, erfindungsgemäß bevorzugt.

Es ist vorteilhaft im Sinne der vorliegenden Erfindung, wenn die Zubereitung Ethanol in einer Konzentration von 0,5 bis 10 Gewichts-%, bezogen auf das Gesamtgewicht der Zubereitung, enthält. Dabei wird ein Gehalt von 4 bis 10 Gewichts-%, bezogen auf das Gesamtgewicht der Zubereitung, erfindungsgemäß bevorzugt.

Erfindungsgemäß vorteilhafte Ausführungsformen der vorliegenden Erfindung sind dadurch gekennzeichnet, dass die Zubereitung das Titandioxid in der Kristallstruktur Rutil mit einer Primärpartikelgröße von 2 -100 nm in einer Konzentration von 0,5 bis 10 Gewichts-%, bezogen auf das Gesamtgewicht der Zubereitung, enthält. Dabei wird ein Gehalt von 1 bis 6 Gewichts-%, bezogen auf das Gesamtgewicht der Zubereitung, erfindungsgemäß bevorzugt.

Die erfindungsgemäße kosmetische Zubereitung kann in unterschiedlichen Formen vorliegen, beispielsweise in Form einer Emulsion oder Hydrodispersion. Dabei ist es erfindungsgemäß vorteilhaft, wenn die Zubereitung in Form einer Emulsion vorliegt. Erfindungsgemäß bevorzugt ist es dabei, wenn die Zubereitung in Form einer O/W-Emulsion (Öl-in-Wasser-Emulsion) vorliegt.

Liegt die erfindungsgemäße kosmetische Zubereitung in Form einer Emulsion (insbesondere O/W-Emulsion) vor, so ist es erfindungsgemäß vorteilhaft, wenn die Zubereitung einen oder mehrere Emulgatoren enthält, die gewählt werden aus der Gruppe der Verbindungen Glycerylstearatcitrat, Glycerylstearat (selbstemulgierend), Stearinsäure, Stearatsalze, Polyglyceryl-3-methylglycosedistearat, Natriumcetearylsulfat, Natriumstaroylglutamat, Polyglyceryl-10 stearat, Cetearylalkohol, Cetearylsulfosuccinat, Kaliumcetylphosphat, enthält. Erfindungsgemäß bevorzugt sind dabei die Emulgatoren Glycerylstearat (selbstemulgierend), Stearinsäure, Stearatsalze, Polyglyceryl-3-methylglycosedistearat, Natriumcetearylsulfat, Natriumstaroylglutamat,

Polyglyceryl-10 stearat, Cetearylalkohol, Cetearylsulfosuccinat, Kaliumcetylphosphat, enthält. Erfindungsgemäß bevorzugt sind dabei die Emulgatoren Glycerylstearat (selbstemulgierend), Stearinsäure, Stearatsalze, Polyglyceryl-3-methylglycosedistearat, Natriumcetearylsulfat, Natriumstaroylglutamat, Polyglyceryl-10 stearat, Cetearylsulfosuccinat.

Erfindungsgemäß vorteilhaft beträgt der Gesamtgehalt dieser Emulgatoren von 0,1 bis 3 Gewichts-%, bezogen auf das Gesamtgewicht der Zubereitung, wobei dieses Gesamtgewicht dem Einzelgewicht eines Emulgators entspricht für den Fall, dass nur eine dieser Verbindungen in der Zubereitung eingesetzt wird.

Ferner zeichnen sich erfindungsgemäß vorteilhafte Ausführungsformen der vorliegenden Erfindung dadurch aus, dass die Zubereitung ein oder mehrere Alkohole gewählt aus der Gruppe der Verbindungen 1,2-Propylenglycol, 1,2-Butylenglycol, 1,3-Butylenglycol, 1,2-Pentandiol, 1,2-Hexandiol, 1,2-Octandiol, 1,2-Decandiol, 2-Methyl-1,3-propandiol Phenoxyethanol und/oder Ethylhexylglycerin enthält, wobei der Einsatz von Ethanol, 1,2-Pentandiol, 1,2-Hexandiol, 1,2-Octandiol, 1,2-Decandiol, 2-Methyl-1,3-propandiol Phenoxyethanol und/oder Ethylhexylglycerin erfindungsgemäß bevorzugt ist. Eine alternative bevorzugte Ausführungsform mit Alkoholen ist die Kombination aus 1,2-Propylenglycol, 1,2-Butylenglycol und/oder 1,3-Butylenglycol mit 1,2-Octandiol.

Erfindungsgemäß vorteilhaft beträgt der Gesamtgehalt dieser Alkohole von 0,1 bis 5 Gewichts-%, bezogen auf das Gesamtgewicht der Zubereitung, wobei dieses Gesamtgewicht dem Einzelgewicht eines Alkohols entspricht für den Fall, dass nur eine dieser Verbindungen in der Zubereitung eingesetzt wird.

Erfindungsgemäß vorteilhafte Ausführungsformen der vorliegenden Erfindung sind auch dadurch gekennzeichnet, dass die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 4-(tert.-Butyl)-4'-methoxydibenzoylmethan; Terephthaliden-dicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; Ethylhexylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan / Dimethylsiloxan - Copolymer; Dioctylbutylamidotriazon (INCI: Diethylhexyl-Butamidotriazone); 2,4-Bis-{[4-(2-ethyl-hexyloxy)-2-hydroxy]-phenyl}-6-(4-methoxyphenyl)-1,3,5-triazin; 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Piperazinderivate; Zinkoxid.

Erfindungsgemäß bevorzugt enthält die Zubereitung 4-(tert.-Butyl)-4'-methoxydibenzoylmethan und/oder 4-(Dimethylamino)-benzoesäure(2-ethylhexyl)ester.

Die erfindungsgemäß vorteilhafte Gesamtkonzentration dieser beiden UV-Filter beträgt von 0,5 bis 5 Gewichts-%, bezogen auf das Gesamtgewicht der Zubereitung, wobei dieses Gesamtgewicht dem Einzelgewicht entspricht für den Fall, dass nur eine dieser Verbindungen in der Zubereitung eingesetzt wird.

Erfindungsgemäß bevorzugt ist es auch, wenn die Zubereitung 2,4,6-Tris-[anilino-(p-carbo-2'-ethyl-1'-hexyloxy)]-1,3,5-triazin (INCI: Ethylhexyl Triazone) und/oder 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol methoxyphenyl Triazine) enthält.

In einem solchen Falle beträgt die erfindungsgemäß vorteilhafte Gesamtkonzentration dieser beiden UV-Filter beträgt von 0,5 bis 8 Gewichts-%, bezogen auf das Gesamtgewicht der Zubereitung, wobei dieses Gesamtgewicht dem Einzelgewicht entspricht für den Fall, dass nur eine dieser Verbindungen in der Zubereitung eingesetzt wird.

Hingegen sind erfindungsgemäß vorteilhafte Ausführungsformen dadurch gekennzeichnet, dass die Emulsion Pirocton-Olamin (1-Hydroxy-4-methyl-6-(2,4,4-trimethylpentyl)-2(1*H*)-pyridon monoethanolaminsalz) enthält.

Enthält die Emulsion Pirocton-Olamin so ist es erfindungsgemäß von Vorteil, wenn der Gehalt an Pirocton-Olamin von 0,01 bis 1 Gew.-%, bezogen auf das Gesamtgewicht der Emulsion beträgt.

Erfindungsgemäß vorteilhafte Ausführungsformen der vorliegenden Erfindung sind dadurch gekennzeichnet, dass die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropyladipat; Diisopropylsebacate, Dicaprylylcarbonat, Di-C12-13 Alkyl Tartrate, Butyloctyl Salicylate, Diethylhexyl Syringylidene Malonate, Hydragenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

Dabei ist es erfindungsgemäß bevorzugt, wenn die Zubereitung Dibutyladipat, Dicaprylylcarbonat und/oder C12-C15 Alkylbenzoat enthält.

Darüber hinaus kann die erfindungsgemäße Zubereitung die üblichen für derartige Zubereitungen bekannten lipophilen Inhaltsstoffe enthalten, beispielsweise Öle, Fette Wachse und dergleichen.

Darüber hinaus ist es erfindungsgemäß von Vorteil, wenn die Zubereitung eine oder mehrere Verbindungen gewählt aus der Gruppe der Verbindungen Gylcyrrhetinsäure, Harnstoff, Arctiin, alpha-Liponsäure, Folsäure, Phytoen, D-Biotin, Coenzym Q10, Hyaluronsäure, alpha-Glucosylrutin, Carnitin, Carnosin, Coffein, natürliche und/oder synthetische Isoflavonoide, Glycerylglucose, Kreatin, Kreatinin, Taurin, ß-Alanin und/oder Licochalcon A, Panthenol, Tocopherol, Tocopherolacetat, Vitamin C, Vitamin C Derivat, Glycyrrhiza Inflata Root Extract, Polydocanol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze, Licochalcon A, enthält.

Die Wasserphase der erfindungsgemäßen Emulsion kann übliche kosmetische Hilfsstoffe enthalten, wie beispielsweise Glycerin, Elektrolyte, Selbstbräuner sowie ein oder mehrere Verdickungsmittel.

Erfindungsgemäß bevorzugt ist es wenn die kosmetische Zubereitung dadurch gekennzeichnet ist, dass die Zubereitung, bezogen auf das Gesamtgewicht der Zubereitung 5 bis 15 Gew.-% Glycerin enthält.

Die erfindungsgemäße Zubereitung kann vorteilhaft Feuchthaltemittel enthalten. Als Feuchthaltemittel (Moisturizer) werden Stoffe oder Stoffgemische bezeichnet, welche kosmetischen Zubereitungen die Eigenschaft verleihen, nach dem Auftragen bzw. Verteilen auf der Hautoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Feuchthaltemittel (Moisturizer) im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zum Schutz vor Hautveränderungen, wie sie z. B. bei der Hautalterung auftreten, verwendet werden.

Die erfindungsgemäßen kosmetischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile® (CAS-Nr. 7631-86-9) und /oder Talkum und/oder Polyethylen, Nylon, Silica Diemthyl Silyate.

Es ist erfindungsgemäß bevorzugt, wenn die erfindungsgemäße Zubereitung Silica Dimethyl Silylate enthält.

Es ist ferner vorteilhaft im Sinne der vorliegenden Erfindung, wenn die erfindungsgemäße Zubereitung einen oder mehrere Parfümstoffe enthält. Diese können beispielsweise vorteilhaft gewählt werden aus der Gruppe der Verbindungen Limonen, Citral, Linalool, alpha-Isomethylionon, Geraniol, Citronellol, 2-Isobutyl-4-hydroxy-4-methyltetrahydropyran, 2-tert-Pentylcyclohexylacetat, 3-Methyl-5-phenyl-1-pentanol, 7-Acetyl-1,1,3,4,4,6-hexamethyltetralin, Adipinsäurediester, alpha-Amylcinnamaldehyd, Alpha-Methylionon, Amyl C Butylphenylmethylpropionalcinnamal, Amylsalicylat, Amylcinnamylalkohol, Anisalkohol, Benzoin, Benzylalkohol, Benzylbenzoat, Benzylcinnamat, Benzylsalicylat, Bergamotöl, bitteres Orangenöl, Butylphenylmethylpropioal, Cardamomöl, Cedrol, Cinnamal, Cinnamylalkohol, Citronellylmethylcrotonat, Citronenöl, Coumarin, Diethylsuccinat, Ethyllinalool, Ethylenbrassylat, Eugenol, Evernia Furfuracea Extract, Evernia Prunastri Extract, Farnesol, Guajakholzöl, Hexylcinnamal, Hexylsalicylat, Hydroxycitronellal, Lavendelöl, Lemonenöl, Linaylacetat, Mandarinenöl, Menthyl PCA, Methylheptenon, Muskatnussöl, Rosmarinöl, süßes Orangenöl, Terpineol, Tonkabohnenöl, Triethylcitrat und/oder Vanillin.

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im Wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 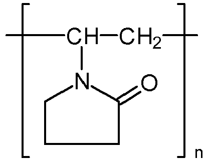 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79), Acrylates Copolymer (Epitex 66). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Die erfindungsgemäße Zubereitung kann vorteilhaft als Creme, Lotion, Spray oder Stift-förmige Zubereitung dargereicht werden.

Erfindungsgemäß ist ihr Einsatz als Tagespflegeprodukt oder Sonnenschutzmittel.

Erfindungsgemäß ist nicht zuletzt die Verwendung des erfindungsgemäßen Titandioxides in der Kristallstruktur Rutil mit einer Primärpartikelgröße von 2 -100 nm zur Reduzierung der geruchlichen Fehlnote von Sotolon in kosmetischen Zubereitungen enthaltend Octocrylen und Ethanol sowie die die Verwendung des erfindungsgemäßen Titandioxides in der Kristallstruktur Rutil mit einer Primärpartikelgröße von 2 -100 nm zur Reduzierung der Gasbildung in kosmetischen Zubereitungen enthaltend Octocrylen, Ethanol und Titandioxid.

### Vergleichsversuch

Mit Hilfe der folgenden Vergleichsversuche konnte der erfinderische Effekt beispielhaft belegt werden:

### Methode:

Kosmetische Formulierungen neigen je nach Rohstoffzusammensetzung zur Bildung der geruchlichen Fehlnote Sotolon. Unter intensiver Lichtbestrahlung kann dieser Vorgang beschleunigt werden und damit eine geruchliche Langzeit-Stabilität vorausgesagt werden.

Die zu untersuchenden Proben (15 ml in 30 ml Glasbehältern) werden im Suntester (Suntest CPLS+, Firma Atlas) 60h bestrahlt (Einstellung: 765 W/m², UV Filter Nr. 56052317, Coated Quarz Glass). Diese Bestrahlung simuliert eine intensive Lichtbelastung.

Anschließend werden die Muster auf einer Skala von 1 - 6 geruchlich bewertet. Dabei bedeutet 1=sehr gut und 6=sehr schlecht.

| | **PMCCBB30 290** | **PMCCBB30 295** | **PMCCBB30 296** |
|---|---|---|---|
| **INCI** | **m [%]** | **m [%]** | **m [%]** |
| Tocopheryl Acetate | 0,06 | 0,06 | 0,06 |
| Panthenol | 1,00 | 1,00 | 1,00 |
| Ethylhexylglycerin | 0,30 | 0,30 | 0,30 |
| Hydrogenated Coco-Glycerides | 1,00 | 1,00 | 1,00 |
| Myristyl Myristate | 1,00 | 1,00 | 1,00 |
| C12-15 Alkyl Benzoate | 4,50 | 4,50 | 4,50 |
| Glyceryl Stearate Citrate | 2,00 | 2,00 | 2,00 |
| VP/Hexadecene Copolymer | 0,50 | 0,50 | 0,50 |
| Parfum | 0,40 | 0,40 | 0,40 |
| Glycerin | 8,50 | 8,50 | 8,50 |
| Citric Acid | 0,09 | 0,09 | 0,09 |
| Sodium Citrate | 0,17 | 0,17 | 0,17 |
| Aqua + Sodium Hydroxide | 0,05 | 0,05 | 0,05 |
| Ethylparaben | 0,20 | 0,20 | 0,20 |
| Methylparaben | 0,30 | 0,30 | 0,30 |
| Phenoxyethanol | 0,50 | 0,50 | 0,50 |
| Cetyl Alcohol | 0,50 | 0,50 | 0,50 |
| Xanthan Gum | 0,30 | 0,30 | 0,30 |
| Acrylates/C10-30 Alkyl Acrylate Crosspolymer | 0,10 | 0,10 | 0,10 |
| Stearyl Alcohol | 0,50 | 0,50 | 0,50 |
| Aqua | ad 100 | ad 100 | ad 100 |
| Alcohol Denat. | 4,00 | 4,00 | 4,00 |
| Trisodium EDTA | 0,50 | 0,50 | 0,50 |
| Butyl Methoxydibenzoylmethane | 4,50 | 4,50 | 4,50 |
| Ethylhexyl Salicylate | 4,50 | 4,50 | 4,50 |
| Octocrylene | 9,00 | 9,00 | 9,00 |
| Homosalate | 9,00 | 9,00 | 9,00 |
| Titanium Dioxide + Trimethoxycaprylylsilane (Anatas- Typ) | | | 3,00 |
| Titanium Dioxide + Silica + Dimethicone (Rutil-Typ) | 3,00 | | |
| Titanium Dioxide, Silica, Cetyl Phosphate (Rutil-Typ) | | 3,00 | |
| **Duft-Benotung (1=sehr gut bis 6= sehr schlecht)** | 1 | 2 | 5 |

| | **PMCCBB50 197** | **PMCCBB50 217** | **PMCCBB50 218** |
|---|---|---|---|
| **INCI** | **m [%]** | **m [%]** | **m [%]** |
| Tocopheryl Acetate | 0,06 | 0,06 | 0,06 |
| Panthenol | 1,00 | 1,00 | 1,00 |
| Ethylhexylglycerin | 0,30 | 0,30 | 0,30 |
| Hydrogenated Coco-Glycerides | 1,00 | 1,00 | 1,00 |
| Myristyl Myristate | 1,00 | 1,00 | 1,00 |
| C12-15 Alkyl Benzoate | 4,50 | 4,50 | 4,50 |
| Butylene Glycol Dicaprylate/Dicaprate | 4,50 | 4,50 | 4,50 |
| Dicaprylyl Carbonate | 2,00 | 2,00 | 2,00 |
| Glyceryl Stearate Citrate | 2,00 | 2,00 | 2,00 |
| VP/Hexadecene Copolymer | 0,50 | 0,50 | 0,50 |
| Parfum | 0,40 | 0,40 | 0,40 |
| Glycerin | 8,50 | 8,50 | 8,50 |
| Citric Acid | 0,09 | 0,09 | 0,09 |
| Sodium Citrate | 0,17 | 0,17 | 0,17 |
| Aqua + Sodium Hydroxide | 0,05 | 0,05 | 0,05 |
| Ethylparaben | 0,20 | 0,20 | 0,20 |
| Methylparaben | 0,30 | 0,30 | 0,30 |
| Phenoxyethanol | 0,50 | 0,50 | 0,50 |
| Cetyl Alcohol | 0,50 | 0,50 | 0,50 |
| Xanthan Gum | 0,30 | 0,30 | 0,30 |
| Acrylates/C10-30 Alkyl Acrylate Crosspolymer | 0,10 | 0,10 | 0,10 |
| Stearyl Alcohol | 0,50 | 0,50 | 0,50 |
| Aqua | ad 100 | ad 100 | ad 100 |
| Alcohol Denat. | 4,00 | 4,00 | 4,00 |
| Trisodium EDTA | 0,50 | 0,50 | 0,50 |
| Butyl Methoxydibenzoylmethane | 4,50 | 4,50 | 4,50 |
| Octocrylene | 9,00 | 9,00 | 9,00 |
| Bis-Ethylhexyloxyphenol Methoxyphenyl Triazine | 3,00 | 3,00 | 3,00 |
| Titanium Dioxide + Trimethoxycaprylylsilane (Anatas-Typ) | | | 6,00 |
| Titanium Dioxide + Silica + Dimethicone (Rutil-Typ) | 6,00 | | |
| Titanium Dioxide, Silica, Cetyl Phosphate (Rutil-Typ) | | 6,00 | |
| **Duft-Benotung (1=sehr gut bis 6= sehr schlecht)** | 2 | 3 | 6 |

### Ergebnis:

In den oben aufgeführten kosmetischen Zubereitungen enthaltend Octocrylen und Ethanol kann gezeigt werden, das nur unter Verwendung des erfindungsgemäßen Titandioxides in der Kristallstruktur Rutil eine geruchliche Langzeitstabilität erreicht werden kann. Unter Verwendung des Titandioxides in der Kristallstruktur Anatas bildet sich schnell eine geruchliche Fehlnote.

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| **O/W Lotions** | **1** | **2** | **3** | **4** | **5** |
|---|---|---|---|---|---|
| **INCI** | **m [%]** | **m [%]** | **m [%]** | **m [%]** | **m [%]** |
| Tocopheryl Acetate | 0,06 | 0,10 | 0,10 | 0,15 | 0,50 |
| Panthenol | 1,00 | 1,40 | | 0,50 | 1,00 |
| Ethylhexylglycerin | 0,30 | 0,50 | 0,25 | | |
| 1,2-Hexanediol | | | | 0,40 | |
| Methylpropanediol | | | | | 0,20 |
| Caprylic/Capric Triglyceride | | 4,00 | | 2,00 | |
| Isopropyl Palmitate | | | | | 3,00 |
| Hydrogenated Coco-Glycerides | 1,00 | 2,00 | 9,00 | 1,00 | |
| Dimethicone | | | 1,00 | 0,50 | |
| Octyldodecanol | | | | 2,00 | |
| Ethylhexyl Cocoate | | | | 2,00 | |
| C18-38 Alkyl Hydroxystearoyl Stearate | | | 0,50 | | |
| Cetearyl Alcohol | | | | 0,50 | |
| Myristyl Myristate | 2,00 | 1,00 | 1,50 | | |
| C12-15 Alkyl Benzoate | 4,50 | 6,00 | | | |
| Glyceryl Stearate Citrate | 2,00 | 3,00 | | | |
| Natrium Cetearyl Sulfate | | | 0,15 | | |
| Polyglyceryl-3 Methylglucose Distearate | | | | | 0,15 |
| Glyceryl Stearate SE | | | 1,00 | | |
| Natrium Stearoyl Glutamate | | | | 0,50 | |
| Glyceryl Stearate | | | | 1,00 | |
| Silica Dimethyl Silylate | | 0,50 | 0,50 | 0,30 | 1,00 |
| Tapioca Starch | | | | 3,00 | 3,00 |
| VP/Hexadecene Copolymer | 0,50 | 1,00 | | | |
| Parfum | 0,40 | 0,40 | 0,30 | | 0,20 |
| Glycerin | 6,00 | 8,00 | 5,00 | 1,00 | 1,00 |
| Citric Acid | 0,07 | 0,09 | | | |
| Natrium Citrate | 0,16 | 0,17 | | | |
| Natrium Hydroxide | 0,05 | 0,08 | 0,30 | | 0,20 |
| Ethylparaben | 0,20 | 0,30 | 0,20 | | |
| Methylparaben | 0,20 | 0,30 | 0,30 | | |
| Phenoxyethanol | 0,30 | 0,50 | 0,40 | | 0,50 |
| Cetyl Alcohol | 0,50 | 1,00 | | | |
| Cetearyl Alcohol | | | 1,50 | | |
| Xanthan Gum | 0,30 | 0,50 | 0,40 | 0,30 | |
| Acrylates/C10-30 Alkyl Acrylate Crosspolymer | 0,10 | 0,15 | 0,05 | | 0,20 |
| Carbomer | | | | | 0,15 |
| Hydroxyethylcellulose | | | | | 0,10 |
| Stearyl Alcohol | 0,50 | 0,80 | | | |
| Aqua | ad 100 | ad 100 | ad 100 | ad 100 | ad 100 |
| Alcohol Denat. | 4,00 | 6,00 | 4,00 | 8,00 | 10,00 |
| Trinatrium EDTA | 1,00 | 0,50 | 1,00 | 1,00 | 1,00 |
| Butyl Methoxydibenzoylmethane | 4,50 | 1,50 | 4,75 | 4,00 | 4,00 |
| Ethylhexyl Salicylate | 4,50 | 3,00 | 4,75 | 4,50 | 4,00 |
| Phenylbenzimidazole Sulfonic Acid | | | 1,00 | | |
| Bis-Ethylhexyloxyphenol Methoxyphenyl Triazine | | | 3,50 | | |
| Octocrylene | 9,00 | 5,00 | 9,75 | 9,00 | 8,00 |
| Homosalate | 9,00 | 4,00 | 9,75 | | 8,00 |
| Polysilicone-15 | | | | | 1,00 |
| Titanium Dioxide (nano) + Silica + Dimethicone (Rutil-Typ) | 3,00 | 0,50 | 3,75 | 1,00 | 1,00 |

| **O/W Hydrodispersionen** | **1** | **2** | **3** | **4** |
|---|---|---|---|---|
| **INCI** | **m[%]** | **m[%]** | **m[%]** | **m[%]** |
| Tocopheryl Acetate | 0,10 | 0,10 | 0,50 | 0.50 |
| Panthenol | 1,00 | 1,00 | 1,00 | 0,50 |
| Ethylhexylglycerin | | | 0,25 | 0,30 |
| Dibutyl Adipate | | | | 2,00 |
| 2-Methyl-1,3-propandiol | | | | 0,02 |
| C12-15 Alkyl Benzoate | 9,00 | | | |
| C18-36 Acid Triglyceride | | 1,00 | 1,50 | |
| Butylene Glycol Dicaprylate/Dicaprate | | 2,00 | 3,00 | |
| Polyglyceryl-3 Methylglucose Distearate | 0,20 | | | 0,40 |
| Sucrose Polystearate + Hydrogenated Polyisobutene | | | | 1,00 |
| Sodium Stearoyl Glutamate | | | | |
| Ceteareth-20 | | 1,50 | 1,00 | |
| Silica Dimethyl Silylate | 0,50 | | | |
| Microcrystalline Cellulose + Cellulose Gum | | | | 1,00 |
| VP/Hexadecene Copolymer | 0,50 | 1,00 | 1,00 | 2,00 |
| Parfum | 0,40 | 0,30 | | 0,20 |
| Glycerin | 5,00 | 5,00 | 3,00 | 5,00 |
| Butylene Glycol | | | 4,00 | |
| Natrium Hydroxide | 0,10 | 0,50 | 0,40 | 0,2 |
| Ethylparaben | 0,20 | 0,20 | | |
| Methylparaben | 0,30 | 0,30 | | |
| Phenoxyethanol | 0,50 | 0,50 | | 0,50 |
| Xanthan Gum | 0,05 | | | 0,30 |
| Acrylates/C10-30 Alkyl Acrylate Crosspolymer | 0,20 | | 0,30 | |
| Aqua | ad 100 | ad 100 | ad 100 | ad 100 |
| Alcohol Denat. | 5,00 | 4,00 | 6,00 | 4,00 |
| Trinatrium EDTA | 0,40 | 0,80 | 0,80 | 0,80 |
| Butyl Methoxydibenzoylmethane | 2,50 | 4,50 | 4,50 | 4,75 |
| Phenylbenzimidazole Sulfonic Acid | | 1,50 | 1,00 | 1,00 |
| Ethylhexyl Salicylate | 4,50 | 4,75 | 4,50 | 4,00 |
| Octocrylene | 4,50 | 8,00 | 8,00 | 0,50 |
| Ethylhexyl Triazone | | | | 1,00 |
| Homosalate | | 9,00 | 9,00 | 7,00 |
| Bis-Ethylhexyloxyphenol Methoxyphenyl Triazine | | 3,50 | 3,50 | 3,00 |
| Titanium Dioxide (nano) + Silica + Dimethicone (Rutil-Typ) | 0,50 | 1,00 | 0,80 | 0,50 |

## Patentansprüche

1. Kosmetische Zubereitung enthaltend
a) 2-Ethylhexyl-2-cyano-3,3-diphenylacrylat (Octocrylen),
b) Ethanol und
c) Titandioxid in der Kristallstruktur Rutil mit einer Primärpartikelgröße von 2 -100 nm, wobei die Zubereitung ausschließlich Titandioxid in der Kristallstruktur Rutil enthält, das Titandioxid mit Silica beschichtet ist und das mit Silica beschichtete Titandioxid auf der äußeren Seite der Silica-Schicht eine Schicht aus Dimethicone aufweist, **dadurch gekennzeichnet, dass** die Zubereitung frei ist von Propylparaben, Butylparaben, Methylisothiazolinon, Chlormethylisothiazolinon, IPBC, DMDM-Hydantoin, Dimathylol Glycol, Dimethylol Urea, Sodium Hydroxmethyl Glycinate, BHT, 3-(4-Methylbenzyliden)campher und 2-Hydroxy-4-methoxybenzophenon,

2. Kosmetische Zubereitung nach Anspruch 1, **dadurch gekennzeichnet, dass** die Zubereitung 2-Ethylhexyl-2-cyano-3,3-diphenylacrylat in einer Konzentration von 0,5 bis 9,5 Gewichts-%, bezogen auf das Gesamtgewicht der Zubereitung, enthält.

3. Kosmetische Zubereitung nach einem der vorhergehenden Ansprüche, **dadurch gekennzeichnet, dass** die Zubereitung Ethanol in einer Konzentration von 0,5 bis 10 Gewichts-%, bezogen auf das Gesamtgewicht der Zubereitung, enthält.

4. Kosmetische Zubereitung nach einem der vorhergehenden Ansprüche, **dadurch gekennzeichnet, dass** die sekundäre Partikelgrüße des Titandioxid in der Kristallstruktur Rutil mit einer Primärpartikelgröße von 2 -100 nm zwischen 0,05 und 50 µm beträgt.

5. Kosmetische Zubereitung nach einem der vorhergehenden Ansprüche, **dadurch gekennzeichnet, dass** die Zubereitung das Titandioxid in der Kristallstruktur Rutil mit einer Primärpartikelgröße von 2 -100 nm in einer Konzentration von 0,5 bis 10 Gewichts-%, bezogen auf das Gesamtgewicht der Zubereitung, enthält.

6. Kosmetische Zubereitung nach einem der vorhergehenden Ansprüche, **dadurch gekennzeichnet, dass** die Zubereitung in Form einer Emulsion vorliegt.

7. Kosmetische Zubereitung nach einem der vorhergehenden Ansprüche, **dadurch gekennzeichnet, dass** die Zubereitung einen oder mehrere Emulgatoren gewählt aus der Gruppe der Verbindungen Glycerylstearatcitrat, Glycerylstearat (selbstemulgierend), Stearinsäure, Stearatsalze, Polyglyceryl-3-methylglycosedistearat, Natriumcetearylsulfat, Natriumstaroylglutamat, Polyglyceryl-10 stearat, Cetearylalkohol, Cetearylsulfosuccinat, Kaliumcetylphosphat, enthält

8. Kosmetische Zubereitung nach einem der vorhergehenden Ansprüche, **dadurch gekennzeichnet, dass** die Zubereitung ein oder mehrere Alkohole gewählt aus der Gruppe der Verbindungen 1,2-Pentandiol, 1,2-Hexandiol, 1,2-Octandiol, 1,2-Decandiol, 2-Methyl-1,3-propandiol Phenoxyethanol und/oder Ethylhexylglycerin enthält.

9. Kosmetische Zubereitung nach einem der vorhergehenden Ansprüche, **dadurch gekennzeichnet, dass** die Zubereitung einen oder mehrere UV-Filter enthält, gewählt aus der Gruppe der Verbindungen 2-Phenylbenzimidazol-5-sulfonsäure und/oder deren Salze; Phenylen-1,4-bis-(2-benzimidazyl)-3,3'-5,5'-tetrasulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2hi-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; 4-(tert.-Butyl)-4'-methoxydibenzoylmethan; Terephthaliden-dicamphersulfonsäure; 4-(Dimethylamino)-benzoasäure(2-ethylhexyl)ester: 4-(Dimethylamino)benzoesäure-amylester; 4-Methoxybenzalmalon-säuredi(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester; Homomenthylsalicylat; Ethylhexylsalicylat; 2-Ethylhexyl-2-hydroxybenzoat; Dimethicodiethylbenzalmalonat; 3-(4-(2,2-bis Ethoxycarbonylvinyl)-phenoxy)propenyl)-methoxysiloxan/ Dimethylsiloxan - Copolymer; Dioctylbutylamidotriazon (INCI: Diethylhexyl-Butamidotriazone); 2,4-Bis-{[4-(2-ethyl-hexyloxy)-2-hydroxy]-phenyl}-6-(4-methoxyphenyl)-1,3,5-triazin; 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphonyl-4-yl-1,3,5-triazin; Merocyanine; Piperazinderivate; Zinkoxid.

10. Kosmetische Zubereitung nach einem der vorhergehenden Ansprüche, **dadurch gekennzeichnet, dass** die Zubereitung 2,4,6-Tris-[anilino-(p-carbo-2'-ethyl-1'-hexyloxy)]-1,3,5-triazin (INCI; Ethylhexyl Triazone) und/oder 2,4-Bis-{[4-(2-ethyl-hexyloxy)-2-hydroxy]-phenyl}-6-(4-methoxyphenyl)-1,3,5-triazin (INCI: Bis- Ethylhexyloxyphenol methoxyphenyl Triazine) enthält.

11. Kosmetische Zubereitung nach einem der vorhergehenden Ansprüche, **dadurch gekennzeichnet, dass** die Zubereitung ein oder mehrere Öle gewählt aus der Gruppe der Verbindungen Butylene Glycol Dicaprylat/Dicaprat, Phenethyl Benzoat, C12-15 Alkyl Benzoat, Dibutyladipat; Diisopropyladipat; Diisopropylsebacate, Dicaprylylcarbonat, Di-C12-13 Alkyl Tartrate, Butyloctyl Salicylate, Diethylhexyl Syringylidene Malonate, Hydrogenated Castor Oil Dimerate, Triheptanoin, C12-13 Alkyl Lactate, C16-17 Alkyl Benzoate, Propylheptyl Caprylate, Caprylic/Capric Triglyceride, Diethylhexyl 2,6-Naphthalate, Octyldodecanol, Caprylic/Capric Triglyceride, Ethylhexyl Cocoate, enthält.

12. Kosmetische Zubereitung nach einem der vorhergehenden Ansprüche, **dadurch gekennzeichnet, dass** die Zubereitung eine oder mehrere Verbindungen gewählt aus der Gruppe der Verbindungen Gylcyrrhetinsäure, Harnstoff, Arctiin, alpha-Uponsäure, Folsäure, Phytoen, D-Biotin, Coenzym Q10, Hyaluronsäure, alpha-Glucosylrutin, Carnitin, Carnosin, Coffein, natürliche und/oder synthetische Isoflavonoide, Glycerylglucose, Kreatin, Kreatinin, Taurin, ß-Alanin und/oder Licochalcon A, Panthenol, Tocopherol, Tocopherolacetat, Vitamin C, Vitamin C Derivat, Glycyrrhiza Inflata Root Extract, Polydocanol, Magnolol, Honokiol, Tocopherylacetat, Dihydroxyaceton; 8-Hexadecen-1,16-dicarbonsäure, Glycerylglycose, (2-Hydroxyethyl)harnstoff, Vitamin E bzw. seine Derivate, Hyaluronsäure und/oder deren Salze, Licochalcon A, enthält.

## Claims

1. Cosmetic preparation containing
a) 2-ethylhexyl-2-cyano-3,3-diphenyl acrylate (octocrylene),
b) ethanol and
c) titanium dioxide in the rutile crystal structure with a primary particle size of 2-100 nm, wherein the preparation contains exclusively titanium dioxide in the rutile crystal structure, the titanium dioxide is coated with silica and the titanium dioxide coated with silica has a layer of dimethicone on the exterior side of the silica layer, **characterized in that** the preparation is free of propylparaben, butylparaben, methylisothiazolinone, chloromethylisothiazolinone, IPBC, DMDM hydantoin, dimethylol glycol, dimethylol urea, sodium hydroxymethyl glycinate, BHT, 3-(4-methyl-benzylidene)camphor and 2-hydroxy-4-methoxybenzophenone.

2. Cosmetic preparation according to Claim 1, **characterized in that** the preparation contains 2-ethylhexyl-2-cyano-3,3-diphenyl acrylate in a concentration of 0.5 to 9.5% by weight, based on the total weight of the preparation.

3. Cosmetic preparation according to either of the preceding claims, **characterized in that** the preparation contains ethanol in a concentration of 0.5 to 10% by weight, based on the total weight of the preparation.

4. Cosmetic preparation according to any one of the preceding claims, **characterized in that** the secondary particle size of the titanium dioxide in the rutile crystal structure with a primary particle size of 2-100 nm is between 0.05 and 50 µm.

5. Cosmetic preparation according to any one of the preceding claims, **characterized in that** the preparation contains the titanium dioxide in the rutile crystal structure with a primary particle size of 2-100 nm in a concentration of 0.5 to 10% by weight, based on the total weight of the preparation.

6. Cosmetic preparation according to any one of the preceding claims, **characterized in that** the preparation is present in the form of an emulsion.

7. Cosmetic preparation according to any one of the preceding claims, **characterized in that** the preparation contains one or more emulsifiers selected from the group of compounds glyceryl stearate citrate, glyceryl stearate (self-emulsifying), stearic acid, stearate salts, polyglyceryl-3-methylglycose distearate, sodium cetearyl sulfate, sodium stearoyl glutamate, polyglyceryl-10 stearate, cetearyl alcohol, cetearyl sulfosuccinate, potassium cetyl phosphate.

8. Cosmetic preparation according to any one of the preceding claims, **characterized in that** the preparation contains one or more alcohols selected from the group of compounds 1,2-pentanediol, 1,2-hexanediol, 1,2-octanediol, 1,2-decanediol, 2-methyl-1,3-propanediol, phenoxyethanol and/or ethyl hexyl glycerin.

9. Cosmetic preparation according to any one of the preceding claims, **characterized in that** the preparation contains one or more UV filters selected from the group of compounds 2-phenylbenzimidazole-5-sulfonic acid and/or the salts thereof; phenylene-1,4-bis-(2-benzimidazyl)-3,3'-5,5'-tetrasulfonic acid salts; 1,4-di(2-oxo-10-sulfo-3-bornylidenemethyl)benzene and salts thereof; 4-(2-oxo-3-bornylidenemethyl)benzene sulfonic acid salts; 2-methyl-5-(2-oxo-3-bornylidenemethyl)sulfonic acid salts; 2,2'-methylene-bis-(6-(2H-benzotriazol-2-yl)-4-(1,1,3,3-tetramethylbutyl)phenol); 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methyl-benzylidene)camphor; 3-benzylidenecamphor; 4-(tert-butyl)-4'-methoxydibenzoylmethane; terephthalidene dicamphor sulfonic acid; 4-(dimethylamino)benzoic acid (2-ethylhexyl)ester; 4-(dimethylamino)benzoic acid amyl ester; 4-methoxybenzalmalonic acid di(2-ethylhexyl)ester; 4-methoxycinnamic acid isoamyl ester; 2-hydroxy-4-methoxybenzophenone, 2-hydroxy-4-methoxy-4'-methylbenzophenone; 2,2'-dihydroxy-4-methoxybenzophenone; 2-(4'-diethylamino-2'-hydroxybenzoyl)benzoic acid hexyl ester; homomenthyl salicylate; ethylhexyl salicylate; 2-ethylhexyl-2-hydroxybenzoate; dimethicodiethylbenzalmalonate; 3-(4-(2,2-bis-ethoxycarbonylvinyl)phenoxypropenyl)methoxysiloxane/dimethylsiloxane copolymer; dioctylbutylamidotriazone (INCI: diethylhexyl butamidotriazone); 2,4-bis-{[4-(2-ethylhexyloxy)-2-hydroxy]phenyl}-6-(4-methoxyphenyl)-1,3,5-triazine; 2,4-bis-[5-1-(dimethylpropyl)benzoxazol-2-yl-(4-phenyl)imino]-6-(2-ethylhexyl)imino-1,3,5-triazine with the CAS no. 288254-16-0; 4,4',4"-(1,3,5-triazine-2,4,6-triyltriimino)-tris-benzoic acid-tris(2-ethylhexyl ester) (also: 2,4,6-tris-[anilino-(p-carbo-2'-ethyl-1'-hexyloxy)]-1,3,5-triazine (INCI: ethylhexyl triazone); 2,4,6-tribiphenyl-4-yl-1,3,5-triazine; merocyanines; piperazine derivatives; zinc oxide.

10. Cosmetic preparation according to any one of the preceding claims, **characterized in that** the preparation contains 2,4,6-tris-[anilino-(p-carbo-2'-ethyl-1'-hexyloxy)]-1,3,5-triazine (INCI: ethylhexyl triazone) and/or 2,4-bis-{[4-(2-ethylhexyloxy)-2-hydroxy]phenyl}-6-(4-methoxyphenyl)-1,3,5-triazine (INCI: bis-ethylhexyloxyphenol methoxyphenyl triazine).

11. Cosmetic preparation according to any one of the preceding claims, **characterized in that** the preparation contains one or more oils selected from the group of compounds butylene glycol dicaprylate/dicaprate, phenethyl benzoate,C₁₂₋₁₅ alkyl benzoate, dibutyl adipate; diisopropyl adipate; diisopropyl sebacate, dicaprylyl carbonate, di-C₁₂₋₁₃ alkyl tartrate, butyloctyl salicylate, diethylhexyl syringylidene malonate, hydrogenated castor oil dimerate, triheptanoin, C₁₂₋₁₃ alkyl lactate, C₁₆₋₁₇ alkyl benzoate, propylheptyl caprylate, caprylic/capric triglyceride, diethylhexyl 2,6-naphthalate, octyldodecanol, caprylic/capric triglyceride, ethylhexyl cocoate.

12. Cosmetic preparation according to any one of the preceding claims, **characterized in that** the preparation contains one or more compounds selected from the group of compounds glycyrrhetinic acid, urea, arctiin, alpha-lipoic acid, folic acid, phytoene, d-biotine, coenzyme Q10, hyaluronic acid, alpha-glucosyl rutin, carnitine, carnosine, caffeine, natural and/or synthetic isoflavonoids, glyceryl glucose, creatine, creatinine, taurine, β-alanine and/or licochalcone A, panthenol, tocopherol, tocopherol acetate, vitamin C, vitamin C derivative, glycyrrhiza inflata root extract, polydocanol, magnolol, honokiol, tocopheryl acetate, dihydroxyacetone; 8-hexadecene-1,16-dicarboxylic acid, glyceryl glycose, (2-hydroxyethyl)urea, vitamin E and/or its derivatives, hyaluronic acid and/or its salts, licochalcone A.

## Revendications

1. Préparation cosmétique contenant
a) du 2-cyano-3,3-diphénylacrylate de 2-éthylhexyle (octocrylène),
b) de l'éthanol et
c) du dioxyde de titane dans la structure cristalline rutile, présentant une grosseur des particules primaires de 2-100 nm,
la préparation contenant exclusivement du dioxyde de titane dans la structure cristalline rutile, le dioxyde de titane étant revêtu de silice et le dioxyde de titane revêtu de silice présentant, sur le côté externe de la couche de silice, une couche de diméticone, **caractérisée en ce que** la préparation est exempte de propylparabène, de butylparabène, de méthylisothiazolinone, de chlorométhylisothiazolinone, d'IPBC, de DMDM-hydantoïne, de diméthylolglycol, de diméthylolurée, d'hydroxméthylglycinate de sodium, de BHT, de 3-(4-méthylbenzylidène)camphre et de 2-hydroxy-4-méthoxybenzophénone.

2. Préparation cosmétique selon la revendication 1, **caractérisée en ce que** la préparation contient le 2-cyano-3,3-diphénylacrylate de 2-éthylhexyle en une concentration de 0,5 à 9,5% en poids, par rapport au poids total de la préparation.

3. Préparation cosmétique selon l'une quelconque des revendications précédentes, **caractérisée en ce que** la préparation contient de l'éthanol en une concentration de 0,5 à 10% en poids par rapport au poids total de la préparation.

4. Préparation cosmétique selon l'une quelconque des revendications précédentes, **caractérisée en ce que** la grosseur des particules secondaires du dioxyde de titane dans la structure cristalline rutile présentant une grosseur des particules primaires de 2-100 nm est située entre 0,05 et 50 µm.

5. Préparation cosmétique selon l'une quelconque des revendications précédentes, **caractérisée en ce que** la préparation contient le dioxyde de titane dans la structure cristalline rutile présentant une grosseur des particules primaires de 2-100 nm en une concentration de 0,5 à 10% en poids, par rapport au poids total de la préparation.

6. Préparation cosmétique selon l'une quelconque des revendications précédentes, **caractérisée en ce que** la préparation se trouve sous forme d'une émulsion.

7. Préparation cosmétique selon l'une quelconque des revendications précédentes, **caractérisée en ce que** la préparation contient un ou plusieurs émulsifiants, choisis dans le groupe formé par les composés stéarate-citrate de glycéryle, stéarate de glycéryle (auto-émulsifiant), acide stéarique, sels de stéarate, 3-méthylglycosedistéarate de polyglycéryle, cétéarylsulfate de sodium, stéaroylglutamate de sodium, stéarate de polyglycéryl-10, alcool cétéarylique, sulfosuccinate de cétéaryle, cétylphosphate de potassium.

8. Préparation cosmétique selon l'une quelconque des revendications précédentes, **caractérisée en ce que** la préparation contient un ou plusieurs alcools choisis dans le groupe des composés 1,2-pentanediol, 1,2-hexanediol, 1,2-octanediol, 1,2-décanediol, 2-méthyl-1,3-propanediol, phénoxyéthanol et/ou éthylhexylglycérol.

9. Préparation cosmétique selon l'une quelconque des revendications précédentes, **caractérisée en ce que** la préparation contient un ou plusieurs filtres UV, choisis dans le groupe formé par les composés acide 2-phénylbenzimidazole-5-sulfonique et/ou ses sels ; sels de l'acide phénylène-1,4-bis-(2-benzimidazyl)-3,3'-5,5'-tétrasulfonique ; 1,4-di(2-oxo-10-sulfo-3-bornylidèneméthyl)-benzène et ses sels ; sels de l'acide 4-(2-oxo-3-bornylidèneméthyl)benzènesulfonique ; sels de l'acide 2-méthyl-5-(2-oxo-3-bornylidèneméthyl)sulfonique ; 2,2'-méthylène-bis-(6-(2H-benzotriazol-2-yl)-4-(1,1,3,3-tétraméthylbutyl)-phénol) ; 2-(2H-benzotriazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidènecamphre ; 4-(tert-butyl)-4'-méthoxydibenzoylméthane ; acide téréphtalidènedicamphresulfonique ; ester 2-éthylhexylique de l'acide 4-(diméthylamino)-benzoïque ; ester amylique de l'acide 4-(diméthylamino)benzoïque ; ester di-(2-éthylhexylique) de l'acide 4-méthoxybenzalmalonique ; ester isoamylique de l'acide 4-méthoxycinnamique ; 2-hydroxy-4-méthoxybenzophénone, 2-hydroxy-4-méthoxy-4'-méthylbenzophénone ; 2,2'-dihydroxy-4-méthoxybenzophénone ; ester hexylique de l'acide 2-(4'-diéthylamino-2'-hydroxybenzoyl)-benzoïque ; salicylate d'homomenthyle ; salicylate d'éthylhexyle ; 2-hydroxybenzoate de 2-éthylhexyle ; diméthicodiéthylbenzalmalonate ; copolymère de 3-(4-(2,2-bis-éthoxycarbonylvinyl)-phénoxy)propényl)-méthoxysiloxane/diméthylsiloxane ; dioctylbutylamidotriazone (INCI : Diethylhexyl-Butamidotriazone) ; 2,4-bis-{[4-(2-éthylhexyloxy)-2-hydroxy]-phényl}-6-(4-méthoxyphényl)-1,3,5-triazine ; 2,4-bis-[5-1(diméthylpropyl)benzoxazol-2-yl-(4-phényl)-imino]-6-(2-éthylhexyl)-imino-1,3,5-triazine (présentant le n° CAS 288254-16-0) ; ester tris(2-éthylhexylique) de l'acide 4,4',4"-(1,3,5-triazine-2,4,6-triyltriimino)-trisbenzoïque (également : 2,4,6-tris-[anilino-(p-carbo-2'-éthyl-1'-hexyloxy)]-1,3,5-triazine (INCI : Ethylhexyl Triazone)) ; 2,4,6-tribiphényl-4-yl-1,3,5-triazine ; mérocyanines ; dérivés de pipérazine ; oxyde de zinc.

10. Préparation cosmétique selon l'une quelconque des revendications précédentes, **caractérisée en ce que** la préparation contient de la 2,4,6-tris-[anilino-(p-carbo-2'-éthyl-1'-hexyloxy)]-1,3,5-triazine (INCI : Ethylhexyl Triazone) et/ou de la 2,4-bis-{[4-(2-éthylhexyloxy)-2-hydroxy]-phényl}-6-(4-méthoxyphényl)-1,3,5-triazine (INCI : Bis-Ethylhexyloxyphenol Methoxyphenyl Triazine).

11. Préparation cosmétique selon l'une quelconque des revendications précédentes, **caractérisée en ce que** la préparation contient une ou plusieurs huiles choisies dans le groupe des composés dicaprylate/dicaprate de butylèneglycol, benzoate de phénéthyle, benzoate de C12-15-alkyle, adipate de dibutyle ; adipate de diisopropyle, sébacate de diisopropyle, carbonate de dicaprylyle, tartrate de di-C12-13-alkyle, salicylate de butyloctyle, syringylidènemalonate de diéthylhexyle, dimérate d'huile de ricin hydrogénée, triheptanoïne, lactate de C12-13-alkyle, benzoate de C16-17-alkyle, caprylate de propylheptyle, triglycéride caprylique/caprique, 2,6-naphtalate de diéthylhexyle, octyldodécanol, triglycéride caprylique/caprique, cocoate d'éthylhexyle.

12. Préparation cosmétique selon l'une quelconque des revendications précédentes, **caractérisée en ce que** la préparation contient un ou plusieurs composés choisis dans le groupe des composés acide glycyrrhétinique, urée, arctiine, acide alpha-liponique, acide folique, phytoène, D-biotine, coenzyme Q10, acide hyaluronique, alpha-glucosylrutine, carnitine, carnosine, caféine, isoflavonoïdes naturels et/ou synthétiques, glycérylglucose, créatine, créatinine, taurine, β-alanine et/ou licochalcone A, panthénol, tocophérol, acétate de tocophérol, vitamine C, dérivé de vitamine C, extrait de racine de Glycyrrhiza Inflata, polydocanol, magnolol, honokiol, acétate de tocophéryle, dihydroxyacétone ; acide 8-hexadécène-1,16-dicarboxylique, glycérylglycose, (2-hydroxyéthyl)urée, vitamine E ou, selon le cas, ses dérivés, acide hyaluronique et/ou ses sels et/ou licochalcone A.
